(19) Europäisches Patentamt
European Patent Office
Office européen des brevets

(11) **EP 2 238 571 B1**

(12) **EUROPEAN PATENT SPECIFICATION**

(45) Date of publication and mention
of the grant of the patent:
**17.04.2013 Bulletin 2013/16**

(51) Int Cl.:
***G06T 5/00*** (2006.01)

(21) Application number: **08857260.7**

(22) Date of filing: **03.12.2008**

(86) International application number:
**PCT/US2008/085295**

(87) International publication number:
**WO 2009/073672 (11.06.2009 Gazette 2009/24)**

(54) **System for image processing using principal component analysis**

System zur Bildverarbeitung unter Verwendung von Hauptkomponentenanalyse

Système pour traitement d'image par analyse en composantes principales

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **04.12.2007 US 992155 P**

(43) Date of publication of application:
**13.10.2010 Bulletin 2010/41**

(73) Proprietor: **GE Healthcare Limited**
**Little Chalfont**
**Buckinghamshire HP7 9NA (GB)**

(72) Inventors:
• **AXELSSON, Jan**
**S-751 09 Uppsala (SE)**
• **RINGHEIM, Anna**
**05617 Sao Paulo (BR)**

(74) Representative: **Canning, Lewis R.**
**GE Healthcare Limited**
**Pollards Wood**
**Nightingales Lane**
**Chalfont St Giles**
**Buckinghamshire HP8 4SP (GB)**

(56) References cited:
• **RAZIFAR ET AL: "A new application of pre-normalized principal component analysis for improvement of image quality and clinical diagnosis in human brain PET studies" NEUROIMAGE, ACADEMIC PRESS, ORLANDO, FL, US, vol. 33, no. 2, 2 November 2006 (2006-11-02), pages 588-598, XP005732086 ISSN: 1053-8119**
• **PASHA RAZIFAR AND MATS BERGSTRÖM: "13 Application of Multivariate Image Analysis in Nuclear Medicine: Principal Component Analysis (PCA) on Dynamic Human Brain Studies with Positron Emission Tomography (PET) for Discrimination of Areas of Disease at High Noise Levels" TECHNIQUES AND APPLICATIONS OF HYPERSPECTRAL IMAGE ANALYSIS, JOHN WILEY & SONS, LTD, 1 January 2007 (2007-01-01), pages 313-334, XP007906924 ISBN: 978-0-470-01086-0**
• **THIREOU T ET AL: "Performance evaluation of principal component analysis in dynamic FDG-PET studies of recurrent colorectal cancer" COMPUTERIZED MEDICAL IMAGING AND GRAPHICS ELSEVIER UK, vol. 27, no. 1, November 2002 (2002-11), pages 43-51, XP007906963 ISSN: 0895-6111**

**Description**

<u>Field</u>

The present invention relates to image analysis. More particularly, the present invention relates to an improved image analysis technique for medical diagnostics, for example, using positron emission tomography (PET) image data.

<u>Background</u>

Various medical imaging techniques exist to aid clinicians in the diagnosis of pathological conditions caused, for example, by anatomic or functional manifestations of a disease. Many such techniques produce a sequence of image frames that can be used to highlight to the clinician various temporal variations in anatomical and/or functional properties of a patient.

For example, a magnetic resonance imaging (MRI) scan, a functional magnetic resonance imaging (fMRI) scan, a computed tomography (CT) scan, or a single photon emission computed tomography (SPECT) scan can be performed that provides a sequence of image frames showing how a patient's anatomy, such as, for example, the heart or brain, varies over time. Any such temporal variations might be detected during a single scan and/or between multiple scans performed during successive hospital visits, for example.

As another example, PET imaging can be used to obtain a sequence of image frames showing, for example, how the physiological functional properties of a patient's organ, such as, for example, the brain, vary over time.

PET is a known imaging technique that uses tomography to computer-generate a three-dimensional image or map of a functional process in the body as a result of detecting gamma rays when artificially introduced radionuclides incorporated into biochemical substances decay and release positrons. Analysis of the photons detected from the annihilation of these positrons is used to generate the tomographic image frames which may be quantified using a colour scale to show the diffusion of the biochemical substances in the tissue thereby indicating localization of metabolic and/or physiological processes.

For example, radionuclides used in PET may be a short-lived radioactive isotopes such as flourine-18, oxygen-15, nitrogen-13, and carbon-11 (with half-lives ranging from about 110 minutes to about 20 minutes). The radionuclides may be incorporated into biochemical substances such as compounds normally used by the body that may include, for example, sugars, water, and/or ammonia. The biochemical substances may then be injected or inhaled into the body (e.g. into the blood stream) where the substance (e.g. a sugar) becomes concentrated in the tissue of interest, and where the radionuclides decay by emitting positrons. These positrons collide with nearby electrons producing gamma ray photons which can be detected and recorded thereby indicating where the radionuclide was taken up by the body. This set of data may be used to explore and depict one or more of anatomical, physiological, and metabolic information in the human body.

Due to limitations in the amount of radioactivity that can be administered to the subject, a generally short half-life of the radionuclide, and limited sensitivity of certain recording systems, dynamic PET data is typically characterized by a rather high level of noise. A further limiting factor is the metabolic decomposition of the molecule of interest, which lowers the signal and increases the noise. This together with a high level of non-specific binding to the target and the sometimes small differences in target expression between healthy and pathological areas are factors which can make the analysis of dynamic PET data difficult, regardless of the radionuclide used or type of scan conducted. This means that the individual image frames are generally not optimal for the analysis and visualization of anatomy and pathology by a clinician.

Accordingly, various techniques have been developed to try and improve the image quality of the dynamic temporally sequential image frames produced from a PET scan.

One of the standard methods used for the reduction of the noise and quantitative estimation in dynamic PET data is to take the sum, average (or mean) of the image frames of the whole or part of the sequence. However, though sum, average/mean processed images may be effective in reducing noise, this approach results in the dampening of the differences detected between regions with different kinetic behaviour and hence to a reduction in the contrast between non-significant and potentially significant clinical features.

Another method used for analysis of dynamic PET data uses kinetic modelling, with the generation of parametric images, aiming to extract areas with specific kinetic properties that can enhance the discrimination between normal and pathological regions.

One such kinetic modelling method, used for parameter estimation, is known as the Patlak method (or sometimes Gjedde method) [1]. The ratio of target region to reference radioactivity concentration is plotted against a modified time, obtained as the time integral of the reference radioactivity concentration up to the selected time divided by the radioactivity concentration at this time. In cases where the tracer accumulation can be described as irreversible, the Patlak graphical representation of tracer kinetics becomes a straight line with a slope proportional to the accumulation rate. This method can readily be applied to each pixel separately in a dynamic imaging frame sequence and allows the generation of parametric images representative of the accumulation rate.

However, one problem when using kinetic modelling is that the generated parametric images suffer from poor quality, while the images are rather noisy. In this regard, kinetic modelling methods such as the Patlak method [1] do not optimise the signal-to-noise ratio (SNR) during the measurement of physiological parameters from the dynamic data.

Alternative methods for the generation of parametric images also exist, based on other types of modelling. For example, Logan plots, compartment modelling, or extraction of components such as in factor analysis or spectral analysis [2]. Other alternative techniques, such as population based approaches where an iterative two stage (ITS) method is utilized, have also been proposed [3].

Dynamic PET data can also be analyzed using various different multivariate statistical techniques such as principal component analysis (PCA) [4,5, 6, 8, 9, 10]. PCA is employed in order simultaneously to find variance-covariance structures in data in order to reduce the dimensionality of the data set. The results of PCA can be used for different purposes, e.g. factor analysis, regression analysis, performing pre-processing of the input/raw image frame data, etc.

However, in summary, interpretation of sequential image frames remains difficult and there still exists a need for a technique in which clinically significant features can be more reliably extracted for highlighting to clinicians.

Summary of the invention

Various aspects and embodiments of the present invention have thus been devised whilst bearing in mind the aforementioned problems and disadvantages associated with conventional techniques.

According to a first aspect of the present invention, there is provided a method for extracting low-intensity features from an image data set comprising data corresponding to a sequence of original image frames. The method comprises determining a plurality of principal components (PCs) from the image data set corresponding to the original image frames, applying a principal component analysis (PCA) filter to the plurality of principal components to determine a filtered data set by discarding at least one principal component (PC) from the plurality of principal components, and transforming the filtered data set to create a plurality of filtered image frames having enhanced low-intensity features.

The method according to this aspect of the present invention provides various improvements over conventional techniques. For example, it not only enhances low intensity features in the image that might be of clinical significance, but also reduces noise and enables better temporal image visualisation to be provided, for example, when viewing a temporal sequence of image frames such as those depicting tracer uptake during a PET scan where image frame data can be particularly noisy.

According to a second aspect of the present invention, there is provided a computer program product comprising computer code for configuring a data processing apparatus to implement the method according to the first aspect of the present invention.

Such a computer program product may be used, for example, to upgrade the functionality of conventional medical imaging systems to allow them to provide improved image sequences having enhanced low-intensity features.

According to a third aspect of the present invention, there is provided a system for displaying low-intensity features from an image data set comprising data corresponding to a sequence of original image frames. The system comprises an image acquisition module that is operable to acquire the sequence of original image frames. The system also comprises an image analyser that is operable to: a) determine a plurality of principal components from the image data set corresponding to the original image frames, b) apply a principal component analysis (PCA) filter to the plurality of principal components to determine a filtered data set by discarding at least one principal component from the plurality of principal components, and c) transform the filtered data set to create a plurality of filtered image frames having enhanced low-intensity features. The system further comprises a display that is operable to display the filtered image frames, for example, to a clinician for their subsequent interpretation.

It is understood that the elements of such a system may be remotely located from one another, and are not necessarily to be found together in the same physical or geographical location.

Brief description of the drawings

Figure 1 shows a system for clinical diagnosis of a subject according to an embodiment of the present invention;

Figure 2 shows a method for extracting low-intensity features from a dynamic sequential image data set according to various embodiments of the present invention;

Figure 3 shows principal component analysis images derived from an experimental 17 frame dynamic PET brain imaging study conducted in accordance with one aspect of the present invention;

Figure 4 shows filtered frames 11 to 15 from the experimental 17 frame dynamic PET brain imaging study; and

Figure 5 shows original unfiltered frames 11 to 15 from the experimental 17 frame dynamic PET brain imaging study;

Figure 6 shows a first screen shot obtained from a graphical user interface (GUI) for use with various embodiments of the present invention;

Figure 7 shows a further screen shot obtained using the same GUI as Figure 6 but with different operating parameters set; and

Figure 8 shows a screen shot of a residual image obtained using the same GUI as Figure 6 but with different operating parameters set.

<u>Detailed description</u>

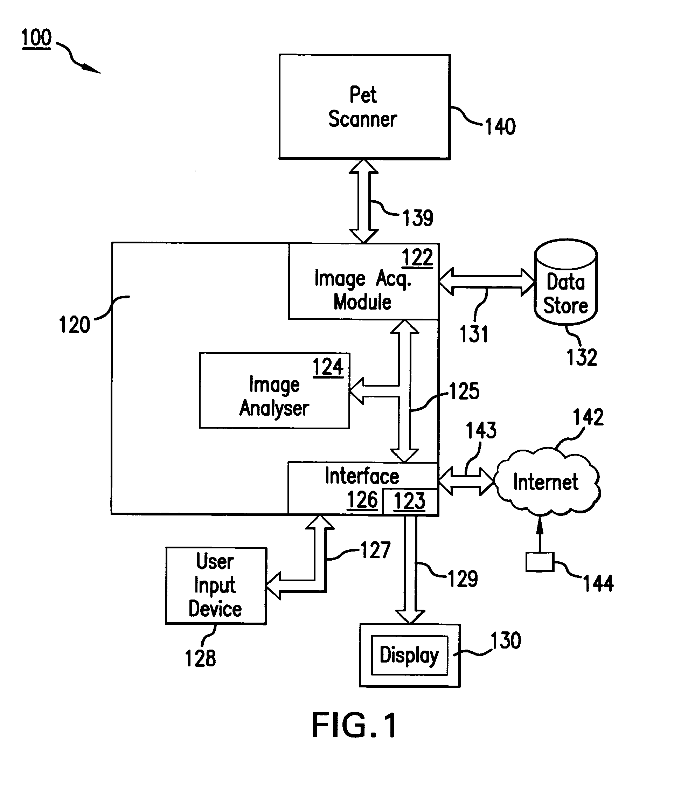

Figure 1 shows a system 100 for clinical diagnosis of a subject according to an embodiment of the present invention. The system 100 includes a data processing apparatus 120 that is configured to provide various interfaces 123,126, an image acquisition module 122 and an image analyser 124. The interfaces 123,126, image acquisition module 122 and image analyser 124 can be logically coupled together by way of a data bus 125 under the control of a central processing unit (not shown).

The data processing apparatus 120 provides a first general purpose interface 126 for interfacing the data processing apparatus 120 to external components. In this embodiment the external components include: an input data link 127 coupled to at least one user input device 128 (e.g. a mouse/keyboard/etc.), a network data link 143 coupled to the Internet 142, and a display data link 129 coupled to a display 130. Additionally, the general purpose interface 126 also provides a GUI 123 through which a user of the system 100 can input data, commands etc., and receive visual information by viewing the display 130.

The GUI 123 may be operable to generate a two- and/or three-dimensional representation of various anatomical portions of the subject. Such representations may, for example, include colour coding of regions according to uptake or use of a substance in respective of those regions. This provides ease of visualisation for users of the system 100. In addition, in various embodiments, a user can also rotate images and/or slice 3D images by manipulating the GUI 123 using the input device 128.

In various embodiments, the data processing apparatus 120 can be provided by a general purpose computer, such as, for example, a personal computer (PC). Such a general purpose computer can use software modules to provide both the image acquisition module 122 and the image analyser 124, and hence can be implemented by upgrading the functional capability of existing equipment using software upgrades. For example, a computer program product 144, comprising computer code, may be transmitted from a remote server (not shown) via the Internet 142 to the data processing apparatus 120 through the network data link 143 or may be provided on a physical medium, such as, for example, a CD, DVD, magnetic disk, ROM, flash memory device, etc.

The system 100 also comprises an optional positron emission tomography (PET) scanner 140 coupled to the data processing apparatus 120 by a data link 139, and an optional data store 132 coupled to the data processing apparatus 120 by a data link 131. The PET scanner 140 and/or the data store 132 may be configured to provide image data to the image acquisition module 122. For example, where no PET scanner is provided, image data could be provided from the data store 132 that may contain previously generated image data stored therein. Such previously generated image data could be generated remotely from the system 100 (e.g. in a remote hospital, etc. where suitable image data generation facilities are available), and subsequently transferred to the data store 132 from where it can be retrieved by the image acquisition module 122. The image acquisition module 122 is further operable to transfer image data generated by the PET scanner 140 to the data store 132 for archiving purposes.

The image analyser 124 is operable to perform image analysis on image data. Such image data can be provided in the form of a sequence of image frames, corresponding, for example, to a temporal sequence of images derived from a certain portion of a subject's anatomy. For example, the image frames may correspond to a time sequence of images showing the uptake of a radio-isotope tagged molecule in a subject's brain, heart, etc. derived from a PET scan. Alternatively, or in addition, the image frames may be derived from magnetic resonance imaging (MRI) (e.g. from different scan sequences, dynamic studies, and/or functional imaging), optical imaging (e.g. at different wavelengths) and/or X-ray imaging (e.g. when performing a dynamic study, CT-scan etc.).

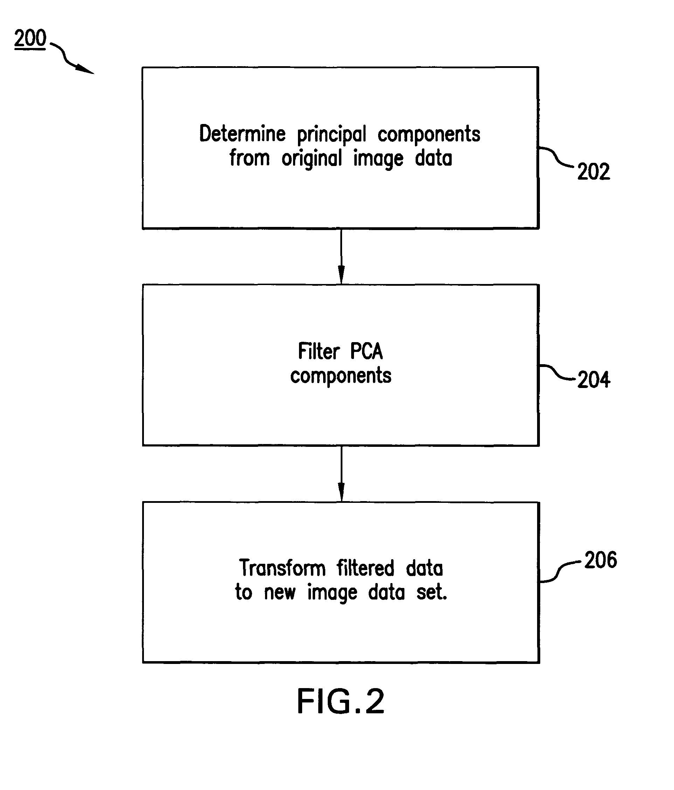

Figure 2 shows a method 200 for extracting low-intensity features from a dynamic sequential image data set according to various embodiments of the present invention. The image data can be in the form of a dynamic sequence of single slices (two-dimensional images) or a dynamic sequence of volumes (three-dimensional, stacks of images handled as one entity). The description below assumes the two-dimensional image sequence. The filtering of the image sequences may be performed for each and every slice location. The method 200 may, for example, be performed using an image analyser 124 as shown in Figure 1, and as described above.

The method comprises a first step 202 of determining the principal components from the data corresponding to each image forming a frame in the sequential image data set.

Pixel value data from each image frame is normalised and put into an *n*-dimensional vector form. The vector is then normalised to a zero mean by subtracting the average data value (e.g. $\bar{x}$, $\bar{y}$, etc.) across each dimension from the data values in that dimension (e.g. $x_i \rightarrow x_i - \bar{x}$, $y_i \rightarrow y_i - \bar{y}$, etc.) and the data is also normalised so that the variance (*var*) is set to unity; wherein the variance (*var*) is defined according to:

$$var = \frac{\sum_{i=1}^{N}\left(X_i - \overline{X}\right)^2}{N} \qquad\qquad - \qquad (1)$$

where $X_i$ is the $i^{th}$ data point in the X dimension, $\overline{X}$ is the mean value of all the data in the X dimension, and $N$ is the total number of data points in the X dimension. Similarly, the data may be normalised by dividing by the standard deviation.

Other normalisation techniques, for example those described by Razifar [6], may prove useful, but the aforementioned method is know to be reliable as it is robust and weights data from all frames equally.

Having normalised the data, including normalising the data sets such that they have a zero mean, principal component analysis (PCA) is applied. In one method for applying PCA, for example as described by Smith [5], a covariance matrix $C^{nxn}$ for a data set having n dimensions is calculated, as follows:

$$cov(X,Y) = \frac{\sum_{i=1}^{N}\left(X_i - \overline{X}\right)\left(Y_i - \overline{Y}\right)}{N} \qquad\qquad - \qquad (2)$$

where covariance is measured between two dimensions, and cov(X,Y) is the covariance measured between the X and Y dimensions. Using equation (2) a covariance matrix can be built up using pairs of data in two dimensions to define the covariance matrix C for a set of data with n dimensions as:

$$C^{nxn} = \left(c_{i,j}, c_{i,j} = cov\left(Dim_i, Dim_j\right)\right) \qquad\qquad - \qquad (3)$$

with $Dim_x$ being the $x^{th}$ dimension. For example, where a three dimensional data set is provided, having dimensions x, y and z, $n = 3$ and the covariance matrix C has three rows and three columns, and is defined as:

$$C = \begin{pmatrix} cov(x,x) & cov(x,y) & cov(x,z) \\ cov(y,x) & cov(y,y) & cov(y,z) \\ cov(z,x) & cov(z,y) & cov(z,z) \end{pmatrix} \qquad\qquad - \qquad (4)$$

Having determined the covariance matrix C, unit eigenvectors for that covariance matrix C are then determined in a conventional manner [4,5].

The eigenvectors thus determined are ordered according to their respective eigenvalues, starting from the eigenvector having the highest eigenvalue (i.e. the most significant component, PC1) and moving to the eigenvector having the lowest eigenvalue (i.e. the least significant component, PCn). The eigenvectors thus ordered PC1-PCn therefore provide a set of n eigenvectors corresponding to the principal components of the image data set for the respective image frame.

Having determined the principal components, the next step in the method 200 is that of applying a PCA filter to the principal components to determine a filtered data set at step 204.

Various techniques can be used to apply the PCA filter so as to discard at least one principal component, and several of these are described further below. By way of definition, as used herein expressions relating to discarding are understood to mean reducing the magnitude of one or more principal components, and as such discarding includes multiplication of one or more principal components by a weighting factor $\alpha$, such that $0 \leq \alpha < 1$.

The principal component for discarding may be a higher order or lower order principal component, the higher order principal components being those grouped towards and including the most significant component PC1, and the lower order principal components being those grouped towards and including the least significant component PCn. Removal of such lower order noise components provides for noise reduction.

In various embodiments, at least one higher order principal component for discarding is determined by merely removing the most significant principal component PC1. For various imaging techniques, PC1 will identify the dynamic behaviour of blood. The removal of one or more higher order principal components may thus be used to remove real features (i.e. not noise) which might otherwise mask fainter features with different kinetic behaviour.

Additionally, or alternatively, at least one principal component for discarding may be determined by dynamically setting one or more variance contribution thresholds and discarding principal components whose percentage variance contribution, e.g. based upon a corresponding eigenvalue, is less than or more than a respective variance contribution threshold.

Various embodiments of the present invention may further use a scree plot analysis, for example, in order to determine one or more lower order principal components for discarding.

A scree plot is a graphical analysis technique with the principal components plotted on the x-axis and a corresponding percentage variance value for each of the principal components plotted on the y-axis. Generally, the scree plot decreases rapidly from the higher order principal components, reaches a "knee", and then levels off.

The scree plot is applied in order to determine where the variance contributions of the principal components (e.g. as defined by respective eigenvalues for the principal components) level off into a noise floor, and discarding those components that are below the noise floor. The noise floor can be determined in various ways, it being data dependent. For example, a lower variance contribution threshold can be set at a value just below the knee of the scree plot with all principal components having a variance value below that threshold being discarded.

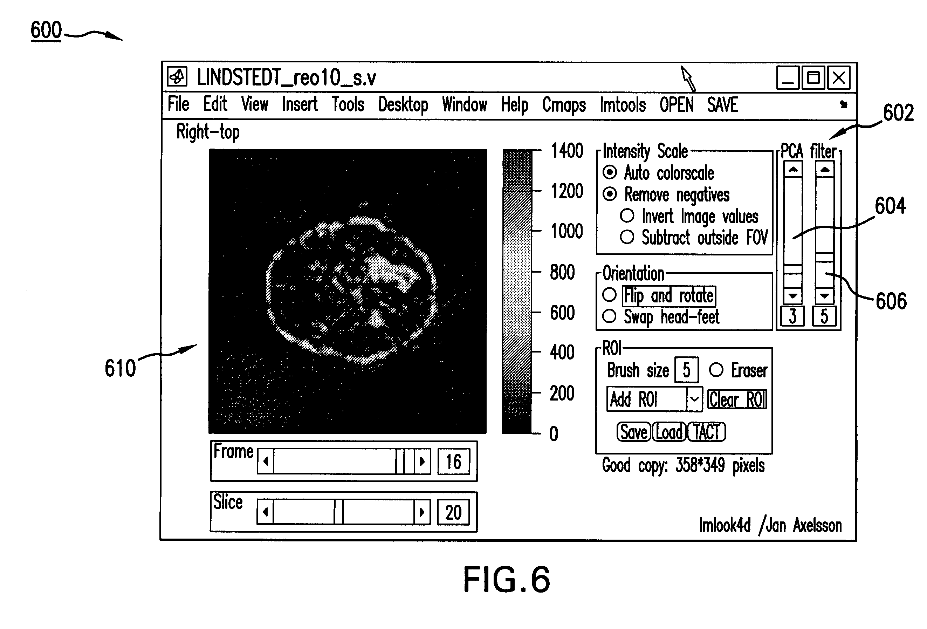

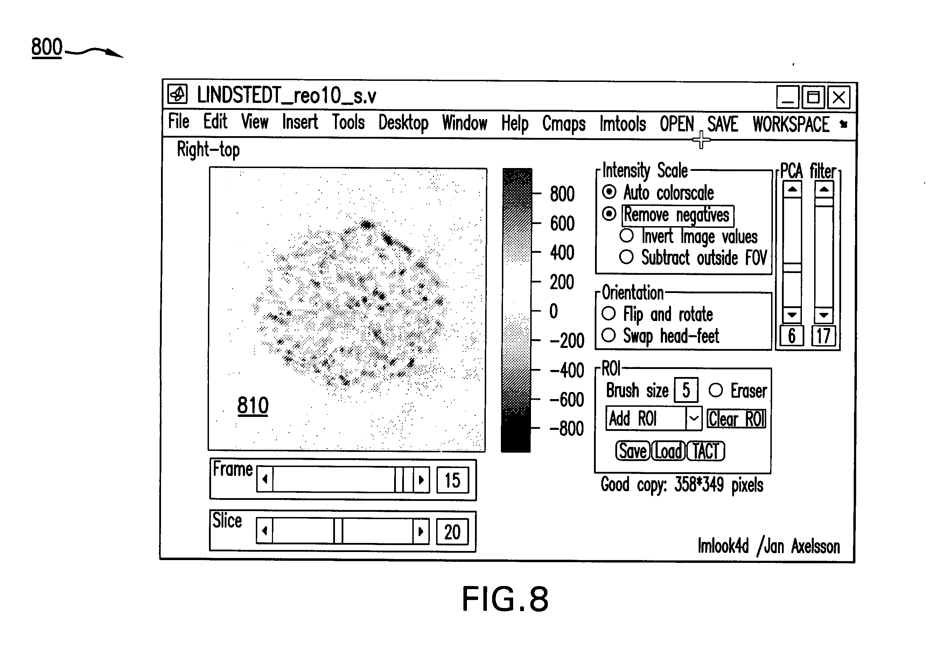

Additionally, or alternatively, at least one principal component for discarding may be determined by dynamically setting one or more principal components to discard, and analyzing the residual of the filtered image. That is, the difference between the filtered and the original image is analyzed, visually or by computer algorithm. The residual image may be calculated using the PCA filter, selecting the lower-order principal components that are discarded (for filtering). That is, the principal components used for creating an image are discarded for the residual image. Figure 8 shows an example of a residual image of the filtered image from Figure 6.

The original image can comprise raw data obtained from a PET scan that corresponds to coincidences between detector pairs. The counts of recorded events for each detector pair is raw data, which can be histogrammed to yield a sinogram. The sinogram can be considered to be an image of counts for each detector pair, and the sinogram can be transformed (e.g. reconstructed) to provide images. In various embodiments, the raw data can thus be filtered prior to transforming, or reconstructing, images.

Various embodiments of methods according to the present invention may further comprise filtering of background pixels from the image data set prior to determining the plurality of principal components.

This is particularly useful for pharmaco-kinetic modelling that uses the input from image frames to calculate significant physiological properties, since it addresses the existing problem that, under certain circumstances, the algorithm used to calculate these properties pixel by pixel is not robust, but instead adds noise to create poor quality images. By performing a filtering step prior to use of such modelling algorithms various embodiments of the present invention address this problem.

For example parametric images may be created using a Patlak model [7]. However, this generates very noisy images. So in order to address this problem, application of a PCA filter according to various embodiments of the present invention is used to remove noise prior to the creation of images using the Patlak technique [7], which in turn results in greatly improved images.

Typically, pharmacological studies require multiple injections of radioactive materials into a subject. Therefore, multiple scans on the same subject (e.g. under different conditions) are presently hampered by a limitation on the maximum permitted radioactivity dose. Hence by using the noise filtering technique of various embodiments of the present invention (e.g. and by removing less significant components only) the number of possible studies on a single person can be usefully extended.

The latter noise filtering technique is also useful when PET scanning is used. For example, it can enable the extraction of better quantative and qualitative information from sequential images acquired by PET scans performed on different parts of the body, without the need to wait for residual radioactivity to decay (which, e.g. with a half-life of almost two hours for flourine-18, would otherwise make this extremely impractical). Such a technique conventionally requires a stepping up of the amount of injected radioactive material, so that the residual signal from each preceding injected dose is masked by the larger signal generated by a subsequently injected dose.

Having applied PCA filter to the principal components to determine a filtered data set at step 204, the next step 206 in the method 200 is that of transforming the filtered data set to a new image data set, *NewDataSet.* The new image data set can be used to provide a plurality of filtered image frames having enhanced low intensity features, that might be of clinical significance, as well as reduced image noise for better visualisation.

The filtered data set is calculated using a feature vector, *FeatureVector.* The feature vector is a matrix of vectors comprising a selection of principal components (eigenvectors), such that:

$$FeatureVector = \left( \alpha_1 PC1 \, \alpha_2 PC2 ... \alpha_n PCn \right) \qquad - \qquad (5)$$

where $\alpha_i$ is the respective weighting factor applied by the PCA filter to the $i^{th}$ eigenvector. A description on how the data is treated for $\alpha_i$ values being 0 or 1 follows. Other $\alpha_i$ values may also be used.

The original frame image data is modified in the following way:

First, image data is rotated to the coordinate system of the principal components (eigenvectors) and the dimensions are truncated to the number of components selected:

$$FinalData = FeatureVector^T \times DataAdjust^T \qquad - \qquad (6a)$$

where *FeatureVector$^T$* is the transpose of *FeatureVector,* and *DataAdjust$^T$* is the transpose of the normalized original image data vectors. Thus, *FinalData* represents PCA-images.

Secondly, the truncated data is transformed back to the original dimensions using the following operation:

$$NewDataSet^T = FeatureVector \times FinalData$$

$$(6b)$$

Thirdly, the normalizations of *NewDataSet* are undone. This is typically done by multiplying by the standard deviation and adding the mean value; e.g. using the standard deviation and mean values being the same values as used above for normalization.

Finally, *NewDataSet* is reformatted to provide images, giving a filtered image set, with the same number of frames as the original image set. This reformatting process can recreate two-dimensional images from the one-dimensional data vectors in *NewDataSet.*

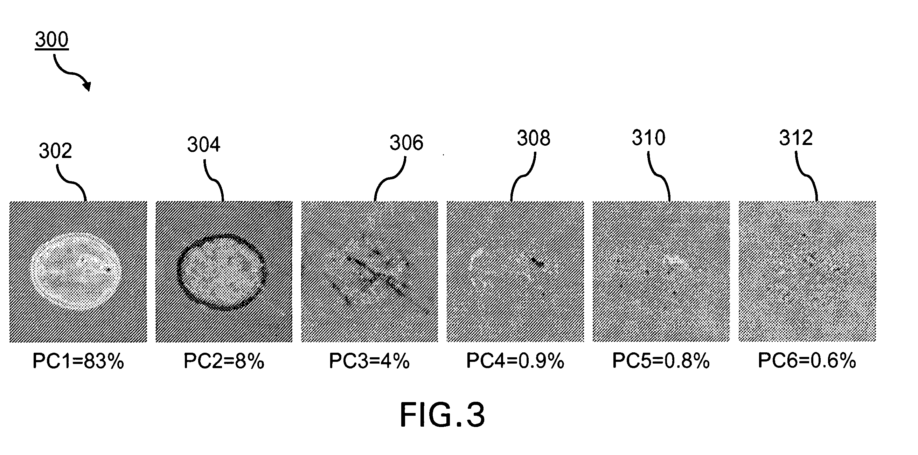

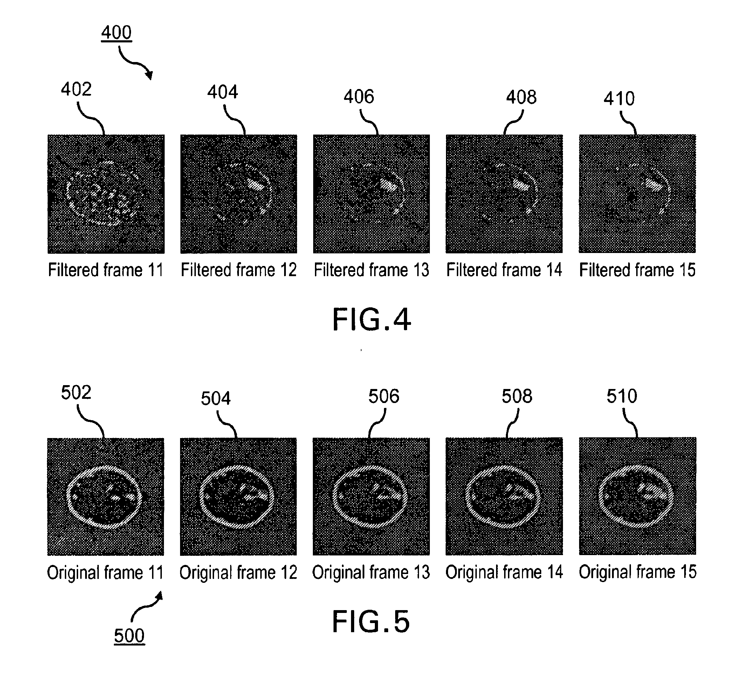

Figure 3 shows principal component analysis images 300 that were derived from an experimental seventeen frame dynamic PET brain imaging study, the eleventh to fifteenth original frames of which are shown in Figure 5.

For this experiment, a General Electric Discovery ST PET/CT camera was used and the tracer was an experimental tracer provided for studying brain damage. The scan times were set to 10 seconds for frames acquired during the first few minutes, with gradually longer acquisition times being used for the frames of up to 15 minutes for the frames acquired after 90 minutes. All the frames were then used in the PCA filtering analysis.

The most significant principal component PC1 contributes 83% to the variance of the principal components. The next highest order principal component PC2 contributes 8% to the variance of the principal components. The third principal component PC3 contributes 4% to the variance of the principal components. The fourth principal component PC4 contributes 0.9% to the variance of the principal components. The fifth principal component PC5 contributes 0.8% to the variance of the principal components. The sixth principal component PC6 contributes 0.6% to the variance of the principal components.

Figure 4 shows filtered frames eleven to fifteen from the experimental seventeen frame dynamic PET brain imaging study. The filtered frames are obtained by applying the method of Figure 2 to the original image frames shown in Figure 5.

In this case, the PCA filter was applied to remove completely the principal components PC1, PC2 and PC7 to PC17 (i.e. $\alpha_1 = \alpha_2 = \alpha_7 = ...\alpha_{17} = 0$). A feature vector was then created in accordance with equation (5) above, and then a new image data set was created from the feature vector in accordance with equations (6a) and (6b).

The displayed subset of the new image data includes the eleventh filtered image frame 402, the twelfth filtered image frame 404, the thirteenth filtered image frame 406, the fourteenth filtered image frame 408, and the fifteenth filtered image frame 410, as shown in Figure 4.

Figure 5 shows original image frames eleven to fifteen from the experimental seventeen frame dynamic PET brain imaging study. Figure 5 is shown adjacent Figure 4 for ease of comparison.

Comparing Figures 4 and 5, it is apparent that a feature of clinical interest is clearly visible in the upper right hand quadrant of the images of filtered image frames twelve to fifteen. This is a low intensity feature that is not readily apparent from a viewing of Figure 5, but which is very clearly delineated with little or no interference being provided as a result of tissue dynamic artefacts. Further studies by the inventors confirmed that the low intensity feature detected as a result of this technique corresponded well with that found using alternative techniques, such as X-ray computer tomography.

Figure 6 shows a first screen shot 600 provided by a GUI in accordance with an embodiment of the present invention. The screen shot 600 may be obtained, for example, from operation of the GUI 123 shown schematically in Figure 1.

The screen shot 600 shows a filtered image frame 610 derived by applying a method in accordance with various aspects of the present invention. The screen shot 600 also shows a PCA filter control section 602 that includes first and second user operable sliders 604, 606.

The first slider 604 can be used to set a first variance contribution threshold for determining which higher order principal components are discarded when creating a filtered data set. In the example case shown, the variance contribution threshold is set to "3" thereby ensuring that the first and second principal components (PC1 and PC2) are discarded when creating the filtered data set.

The second slider 606 can be used to set a second variance contribution threshold for determining which lower order principal components are discarded when creating the filtered data set. In the case shown, the variance contribution threshold is set to "5" ensuring that the third to fifth principal components (PC3 to PC5) are selected for creating the filtered data set with all the other lower order components (in this case PC6 to PC17) being discarded.

The filtered image frame 610 shows an image in which low-intensity features have been enhanced by application of the present invention.

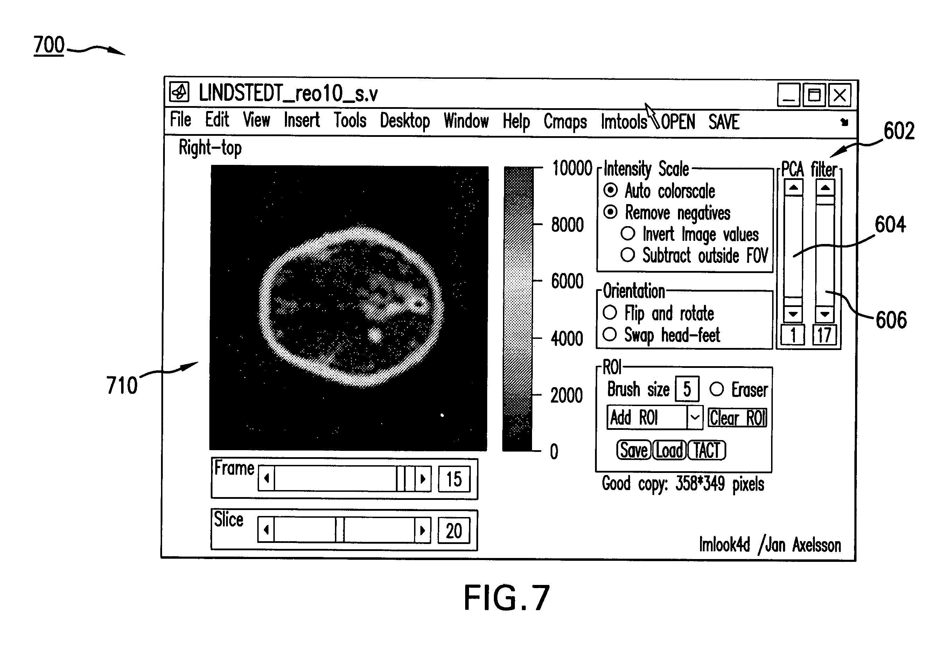

Figure 7 shows a further screen shot 700 obtained from operation of the same GUI as Figure 6 but with different operating parameters having been set.

The screen shot 700 shows an unfiltered image frame 710 derived using all of the principal components determined from a sequence of original image frames.

The screen shot 700 also shows the PCA filter control section 602 including the first and second user operable sliders 604, 606. However, in this case the first slider 604 has been set to "1" thus ensuring that no higher order principal components are discarded when creating a data set for transformation. Additionally, the second slider 606 is set to "17" thereby ensuring that all of the principal components (PC1 to PC17) are selected when creating the data set.

Comparing image frames 610 and 710 from Figures 6 and 7, it is again apparent that features of clinical interest are made more clearly visible by the application of various aspects of the present invention.

It is therefore considered apparent from the inventors' investigations that the hereinmentioned image analysis techniques as provided by various aspects and embodiments of the present invention provide useful improvements over conventional image analysis techniques.

Figure 8 shows a screen shot of a residual image 810 obtained using the same GUI 800 as Figure 6, with different operating parameters set. The residual image 810 is calculated using the PCA filter described above by selecting the lower-order principal components that are discarded (for filtering).

Whilst the present invention has been described in accordance with various aspects and preferred embodiments, it is to be understood that the scope of the invention is not considered to be limited solely thereto and that it is the applicant's intention that all variants and equivalents thereof also fall within the scope of the appended claims.

References

1. A. M. Peters, Graphical Analysis of Dynamic Data: The Patlak-Rutland Plot, Nuclear Medicine Communications, 15:669-672, 1994

2. J. Logan, J. S. Fowler, N. D. Volkow, G-J. Wang, Y-S. Ding, D. L. Alexoff, Distribution Volume Ratios without Blood Sampling from Graphical Analysis of PET Data, Journal of Cerebral Blood Flow Metabolism, 16:834-840, 1986

3. A. Bertoldo, G. Sparacino, C. Cobelli, Population Approach Improves Parameter Estimation of Kinetic Models from Dynamic PET Data, IEEE Transactions on Medical Imaging, vol. 23, no3, pp. 297-306, 2004, ISSN 0278-0062

4. R. C. Gonzalez and R. E. Woods, Digital Image Processing, Second Edition, Chapter 11, Prentice Hall, New Jersey, USA

5. Lindsay I Smith, A tutorial on Principal Components Analysis, 26 February 2002, http://www.cs.otago.ac.nz/cosc453/student_tutorials/principal_components.pdf

6. Pasha Razifar, Novel Approaches for Application of Principal Component Analysis on PET Images for Improvement of Image Quality and Clinical Diagnosis, PhD thesis, Uppsala University, ISSN 1651-6214, ISBN 91-554-6387-8

7. C. S. Patlak and R. G. Blasberg, Graphical Evaluation of Blood-to-Brain Transfer Constants from Multiple-Time Uptake Data, Journal of Cerebral Blood Flow Metabolism, 5:584-590, 1985

8. Razifar et al A new application of pre-normalized principal component analysis for improvement of image quality and clinical diagnosis in human brain PET studies, Neuroimage, 33(2): 588-598, 2006

9. Razifar & Bergström 2007 "Application of Multivariate Image Analysis in Nuclear Medicine: Principal Component Analysis (PCA) on Dynamic Human Brain Studies with Positron Emission Tomography (PET) for Discrimination of Areas of Disease at High Noise Levels" in "Techniques and Applications of Hyperspectral Image Analysis"; John Wiley & Sons: 313-334

10. Thireou et al 2002 "Performance evaluation of principal component analysis in dynamic FDG-PET studies of recurrent colorectal cancer" in "Computerized Medical Imaging and Graphics"; Elsevier: 43-51

**Claims**

1. A system (100) for displaying low-intensity features from an image data set comprising data corresponding to a sequence of original image frames (502, 504, 506, 508, 510), the system (100) comprising:

   (i) an image acquisition module (122) operable to acquire the sequence of original image frames (502, 504, 506, 508, 510);
   (ii) an image analyser (124) operable to:

   a) determine a plurality of principal components (PC1, PC2, PC3, PC4, PC5, PC6) from the image data set corresponding to the original image frames (502, 504, 506, 508, 510),
   b) apply a principal component analysis (PCA) filter to the plurality of principal components (PC1, PC2, PC3, PC4, PC5, PC6) to determine a filtered data set by discarding at least one principal component from the plurality of principal components (PC1, PC2, PC3, PC4, PC5, PC6), and
   c) transform the filtered data set to create a plurality of filtered image frames (402, 404, 406, 408, 410) having enhanced low-intensity features; and

   (iii) a display (130) operable to display the filtered image frames (402, 404, 406, 408, 410),

   wherein the filtered data set is calculated using a feature vector *FeatureVector,* and the feature vector is a matrix of vectors comprising a selection of principal components, such that *FeatureVector* $=(\alpha_1 PC1 \alpha_2 PC2...\alpha_n PCn),$ where $\alpha_i$ is respective weighting factor applied by the principal component analysis filter to the $i^{th}$ principal component *PCi,* wherein step (c) is carried out by: rotating image data to coordinate system of the principal components and truncating dimensions to the number of the selected principal components:

   $$FinalData = FeatureVector^T \times DataAdjust^T$$

   where *FeatureVector$^T$* is the transpose of *Feature Vector,* and *DataAdjust$^T$* is the transpose of the normalized original image data vectors, and *FinalData* represents PCA-images;
   transforming the truncated data back to original dimensions using the following operation:

$$NewDataSet^T = FeatureVector \times FinalData;$$

undoing the normalizations of *NewDataSet* by multiplying by the standard deviation and adding the mean value; reformatting *NewDataSet* to provide images to give a filtered image set with the same number of frames as the original image set.

2. The system (100) of claim 1, wherein at least one said principal component (PC1, PC2, PC3, PC4, PC5, PC6) is a higher order principal component (PC1).

3. The system (100) of claim 2, wherein the image analyser (124) is configured to determine at least one higher order principal component (PC1) for discarding by removing the most significant principal component and/or by dynamically setting a first variance contribution threshold and discarding principal components whose percentage variance contribution to the total principal component variance is less than said first variance contribution threshold.

4. The system (100) of claim 1, wherein at least one said principal component (PC1, PC2, PC3, PC4, PC5, PC6) is a lower order principal component (PC6).

5. The system (100) of claim 4, wherein the image analyser (124) is configured to determine the at least one lower order principal component (PC6) for discarding by dynamically setting a second variance contribution threshold and discarding principal components whose percentage variance contribution to the total principal component variance is less than said second variance contribution threshold and/or by applying a scree plot to determine where the variance contribution of the principal components levels off into a noise floor and discarding those components that are below the noise floor.

6. The system (100) of any one of claims 1-5, wherein the image analyser (124) is further configured to determine at least one said principal component (PC1, PC2, PC3, PC4, PC5, PC6) for discarding by dynamically setting one or more principal components to discard and/or is further configured to determine at least one said principal component (PC1, PC2, PC3, PC4, PC5, PC6) for discarding by analysing one or more residuals of one or more of the filtered image frames (402, 404, 406, 408, 410).

7. The system (100) of any one of claims 1-6, wherein the image analyser (124) is further configured to filter background pixels from the image data set prior to determining the plurality of principal components (PC1, PC2, PC3, PC4, PC5, PC6) from the image data set.

8. The system (100) of any one of claims 1-7, wherein the image analyser (124) is further configured to filter raw data of the original image frames (502, 504, 506, 508, 510) prior to determining (202) the plurality of principal components (PC 1, PC2, PC3, PC4, PC5, PC6) from the raw data set, and to reconstruct a filtered image data set from the filtered raw data.

9. The system (100) of any one of claims 1-8, further comprising a PET scanner (140) operably coupled to the image acquisition module (122).

**Patentansprüche**

1. System (100) zum Anzeigen von Merkmalen geringer Intensität aus einem Bilddatensatz, der Daten umfasst, die einer Sequenz ursprünglicher Einzelbilder (502, 504, 506, 508, 510) entsprechen, wobei das System (100) umfasst:

(i) ein Bilderfassungsmodul (122), das betreibbar ist, um die Sequenz ursprünglicher Einzelbilder (502, 504, 506, 508, 510) zu erfassen;
(ii) eine Bildanalysevorrichtung (124), die betreibbar ist, um:

a) mehrere Hauptkomponenten (PC1, PC2, PC3, PC4, PC5, PC6) aus dem Bilddatensatz zu bestimmen, der den ursprünglichen Einzelbildern (502, 504, 506, 508, 510) entspricht,
b) einen Hauptkomponentenanalyse-(PCA: Principal Component Analysis)-Filter auf die mehreren Hauptkomponenten (PC1, PC2, PC3, PC4, PC5, PC6) anzuwenden, um einen gefilterten Datensatz zu bestimmen durch Verwerfen zumindest einer Hauptkomponente aus den mehreren Hauptkomponenten (PC1, PC2, PC3, PC4, PC5, PC6), und

c) den gefilterten Datensatz zu transformieren, um mehrere gefilterte Einzelbilder (402, 404, 406, 408, 410) mit verstärkten Merkmalen geringer Intensität zu erstellen; und

(iii) eine Anzeige (130), die betreibbar ist, um die gefilterten Einzelbilder (402, 404, 406, 408, 410) anzuzeigen,

wobei der gefilterte Datensatz berechnet wird unter Verwendung eines Merkmalsvektors *FeatureVector,* und der Merkmalsvektor eine Matrix von Vektoren, umfassend eine Auswahl von Hauptkomponenten, ist, so dass *Feature-Vector = ($\alpha_1$PC1$\alpha_2$PC2 ... $a_n$PCn),* worin $\alpha_i$ für einen jeweiligen Gewichtungsfaktor steht, der durch den Hauptkomponentenanalysefilter auf die $i^{te}$ Hauptkomponente *PCi* angewandt wird,

wobei Schritt (c) ausgeführt wird durch: Rotieren von Bilddaten bezüglich des Koordinatensystems der Hauptkomponenten, und Reduzieren von Dimensionen auf die Anzahl der ausgewählten Hauptkomponenten:

$$FinalData = FeatureVector^T \times DataAdjust^T$$

worin *FeatureVector$^T$* für die Transponierte von *FeatureVector* steht, und *DataAdjust$^T$* für die Transponierte der normalisierten Vektoren der ursprünglichen Bilddaten steht, und *FinalData* PCA-Bilder darstellt;

Transformieren der reduzierten Daten zurück zu ursprünglichen Dimensionen mittels der folgenden Operation:

$$NewDataSet^T = FeatureVector \times FinalData;$$

Rückgängigmachen der Normalisierungen von *NewDataSet* durch Multiplizieren mit der Standardabweichung und Addieren des Mittelwerts;

Reformatieren von *NewDataSet,* um Bilder bereitzustellen, die einen gefilterten Bildsatz mit der gleichen Anzahl von Einzelbildern wie der ursprüngliche Bildsatz ergeben.

2. System (100) nach Anspruch 1, wobei zumindest eine Hauptkomponente (PC1, PC2, PC3, PC4, PC5, PC6) eine Hauptkomponente höherer Ordnung (PC1) ist.

3. System (100) nach Anspruch 2, wobei die Bildanalysevorrichtung (124) ausgebildet ist, um zumindest eine Hauptkomponente höherer Ordnung (PC1) zwecks Verwerfung zu bestimmen durch Entfernen der höchstwertigen Hauptkomponente und/oder durch dynamisches Festlegen einer ersten Varianzbeitragsschwelle und Verwerfen von Hauptkomponenten, deren prozentualer Varianzbeitrag zur gesamten Hauptkomponentenvarianz niedriger als die erste Varianzbeitragsschwelle ist.

4. System (100) nach Anspruch 1, wobei zumindest eine Hauptkomponente (PC1, PC2, PC3, PC4, PC5, PC6) eine Hauptkomponente niedrigerer Ordnung (PC6) ist.

5. System (100) nach Anspruch 4, wobei die Bildanalysevorrichtung (124) ausgebildet ist, um die zumindest eine Hauptkomponente niedrigerer Ordnung (PC6) zwecks Verwerfung zu bestimmen durch dynamisches Festlegen einer zweiten Varianzbeitragsschwelle und Verwerfen von Hauptkomponenten, deren prozentualer Varianzbeitrag zur gesamten Hauptkomponentenvarianz niedriger als die zweite Varianzbeitragsschwelle ist, und/oder durch Anwenden eines Scree-Plots, um zu bestimmen, wo sich der Varianzbeitrag der Hauptkomponenten auf ein Grundrauschen einpendelt, und Verwerfen jener Komponenten, die unterhalb des Grundrauschens liegen.

6. System (100) nach einem der Ansprüche 1 - 5, wobei die Bildanalysevorrichtung (124) weiterhin ausgebildet ist, um zumindest eine Hauptkomponente (PC1, PC2, PC3, PC4, PC5, PC6) zwecks Verwerfung zu bestimmen durch dynamisches Festlegen einer oder mehrerer Hauptkomponenten zum Verwerfen, und/oder weiterhin ausgebildet ist, um zumindest eine Hauptkomponente (PC1, PC2, PC3, PC4, PC5, PC6) zwecks Verwerfung zu bestimmen durch Analysieren eines oder mehrerer Reste von einem oder mehreren der gefilterten Einzelbilder (402, 404, 406, 408, 410).

**7.** System (100) nach einem der Ansprüche 1 - 6, wobei die Bildanalysevorrichtung (124) weiterhin ausgebildet ist, um Hintergrundbildpunkte aus dem Bilddatensatz zu filtern vor Bestimmung der mehreren Hauptkomponenten (PC1, PC2, PC3, PC4, PC5, PC6) aus dem Bilddatensatz.

**8.** System (100) nach einem der Ansprüche 1 - 7, wobei die Bildanalysevorrichtung (124) weiterhin ausgebildet ist, um Rohdaten der ursprünglichen Einzelbilder (502, 504, 506, 508, 510) zu filtern vor Bestimmung (202) der mehreren Hauptkomponenten (PC1, PC2, PC3, PC4, PC5, PC6) aus dem Rohdatensatz, und um einen gefilterten Bilddatensatz aus den gefilterten Rohdaten zu rekonstruieren.

**9.** System (100) nach einem der Ansprüche 1 - 8, das weiterhin einen PET-Scanner (140) umfasst, der an das Bilderfassungsmodul (122) funktionell gekoppelt ist.

**Revendications**

**1.** Dispositif (100) destiné à afficher des particularités de faible intensité à partir d'un ensemble de données d'image comprenant des données correspondant à une séquence de trames d'image initiales (502, 504, 506, 508, 510), le dispositif (100) comprenant :

(i) un module d'acquisition d'image (122) pouvant être utilisé de manière à acquérir la séquence de trames d'image initiales (502, 504, 506, 508, 510) ;
(ii) un analyseur d'image (124) pouvant être utilisé de manière à :

a) déterminer une pluralité de composantes principales (PC1, PC2, PC3, PC4, PC5, PC6) à partir de l'ensemble de données d'image correspondant aux trames d'image initiales (502, 504, 506, 508, 510),
b) appliquer un filtre d'analyse de composante principale (PCA) sur la pluralité de composantes principales (PC1, PC2, PC3, PC4, PC5, PC6) afin de déterminer un ensemble de données filtrées en rejetant au moins une composante principale de la pluralité de composantes principales (PC1, PC2, PC3, PC4, PC5, PC6), et
c) transformer l'ensemble de données filtrées afin de créer une pluralité de trames d'image filtrées (402, 404, 406, 408, 410) présentant des particularités de faible intensité améliorées ; et

(iii) un dispositif d'affichage (130) pouvant être utilisé de manière à afficher les trames d'image filtrées (402, 404, 406, 408, 410),

dans lequel l'ensemble de données filtrées est calculé en utilisant un vecteur de particularité "Vecteur_Particularité", et le vecteur de particularité est une matrice de vecteurs comprenant une sélection de composantes principales, de telle sorte que Vecteur_Particularité = $(a_1.PC1\ a_2\ PC2... a_nPCn)$, où $a_i$ est un facteur de pondération respectif appliqué par le filtre d'analyse de composante principale sur la $i^{ème}$ composante principale PCi,
dans lequel l'étape (c) est mise en oeuvre par :
rotation des données d'image sur un système de coordonnées des composantes
principales et troncature de dimensions sur le nombre des composantes principales sélectionnées :

$$\text{Données\_Finales} = \text{Vecteur\_Particularité}^T \times \text{Ajustement\_Données}^T$$

où $\text{Vecteur\_Particularité}^T$ représente la transposée de Vecteur_Particularité, et $\text{Ajustement\_Données}^T$ représente la transposée des vecteurs de données d'image initiales normalisés, et Données_Finales représente les images de PCA;
transformation des données tronquées afin de revenir aux dimensions initiales en utilisant l'opération suivante :

$$\text{Nouvel\_Ensemble\_Données}^T = \text{Vecteur\_Particularité} \times \text{Données\_Finales} ;$$

annulation des normalisations de Nouvel_Ensemble_Données par multiplication par l'écart type et ajout de la valeur moyenne ;
reformatage de Nouvel_Ensemble_Données afin de fournir des images permettant d'obtenir un ensemble d'image filtré avec le même nombre de trames que l'ensemble d'image initial.

2. Dispositif (100) selon la revendication 1, dans lequel au moins l'une desdites composantes principales (PC1, PC2, PC3, PC4, PC5, PC6) est une composante principale d'ordre supérieur (PC1).

3. Dispositif (100) selon la revendication 2, dans lequel l'analyseur d'image (124) est configuré afin de déterminer au moins une composante principale d'ordre supérieur (PC1) à rejeter par élimination de la composante principale la plus significative et/ou initialisation dynamique d'un premier seuil de contribution de variance et rejet des composantes principales dont le pourcentage de contribution de variance à la variance de composante principale totale est inférieure audit premier seuil de contribution de variance.

4. Dispositif (100) selon la revendication 1, dans lequel au moins l'une desdites composantes principales (PC1, PC2, PC3, PC4, PC5, PC6) est une composante principale d'ordre inférieur (PC6).

5. Dispositif (100) selon la revendication 4, dans lequel l'analyseur d'image (124) est configuré afin de déterminer la ou les composantes principales d'ordre inférieur (PC6) à rejeter par initialisation dynamique d'un second seuil de contribution de variance et rejet des composantes principales dont le pourcentage de contribution de variance à la variance de composante principale totale est inférieur au second seuil de contribution de variance et/ou par application d'un graphe des valeurs propres ordonnées afin de déterminer où la contribution de variance des composantes principales se stabilise à un seuil de bruit et rejet des composantes qui sont au-dessous du seuil de bruit.

6. Dispositif (100) selon l'une quelconque des revendications 1 à 5, dans lequel l'analyseur d'image (124) est, en outre, configuré afin de déterminer au moins une desdites composantes principales (PC1, PC2, PC3, PC4, PC5, PC6) à rejeter par initialisation dynamique d'une ou plusieurs des composantes principales à rejeter et/ou est, en outre, configuré afin de déterminer au moins une desdites composantes principales (PC1, PC2, PC3, PC4, PC5, PC6) à rejeter par analyse d'un ou plusieurs résidus d'une ou plusieurs des trames d'image filtrées (402, 404, 406, 408, 410).

7. Dispositif (100) selon l'une quelconque des revendications 1 à 6, dans lequel l'analyseur d'image (124) est, en outre, configuré afin de filtrer des pixels d'arrière-plan à partir de l'ensemble de données d'image avant détermination de la pluralité de composantes principales (PC1, PC2, PC3, PC4, PC5, PC6) à partir de l'ensemble de données d'image.

8. Dispositif (100) selon l'une quelconque des revendications 1 à 7, dans lequel l'analyseur d'image (124) est, en outre, configuré afin de filtrer les données brutes des trames d'image initiales (502, 504, 506, 508, 510) avant détermination (202) de la pluralité des composantes principales (PC1, PC2, PC3, PC4, PC5, PC6) à partir de l'ensemble de données brutes, et reconstruction de l'ensemble des données d'image filtrées à partir des données brutes filtrées.

9. Dispositif (100) selon l'une quelconque des revendications 1 à 8, comprenant, en outre, un scanneur de tomographie TEP (140) couplé de manière opérationnelle au module d'acquisition d'image (122).

100

Pet
Scanner — 140

139

120

Image Acq. **122**
Module

Data
Store

131

132

Image **124**
Analyser

125

143 142

Interface
**126** **123**

Internet

127

129

144

User
Input
Device

Display — 130

128

# FIG.1

200

```
┌─────────────────────────────────┐
│                                 │
│   Determine principal components│  202
│   from original image data      │
│                                 │
└─────────────────────────────────┘
                 │
                 ▼
┌─────────────────────────────────┐
│                                 │
│        Filter PCA               │  204
│        components               │
│                                 │
└─────────────────────────────────┘
                 │
                 ▼
┌─────────────────────────────────┐
│                                 │
│   Transform filtered data       │  206
│   to new image data set.        │
│                                 │
└─────────────────────────────────┘
```

# FIG.2

FIG.3

EP 2 238 571 B1

400

402    404    406    408    410

Filtered frame 11   Filtered frame 12   Filtered frame 13   Filtered frame 14   Filtered frame 15

# FIG.4

502    504    506    508    510

Original frame 11   Original frame 12   Original frame 13   Original frame 14   Original frame 15

500

# FIG.5

FIG.6

FIG.7

EP 2 238 571 B1

FIG.8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **A. M. PETERS.** Graphical Analysis of Dynamic Data: The Patlak-Rutland Plot. *Nuclear Medicine Communications,* 1994, vol. 15, 669-672
- **J. LOGAN ; J. S. FOWLER ; N. D. VOLKOW ; G-J. WANG ; Y-S. DING ; D. L. ALEXOFF.** Distribution Volume Ratios without Blood Sampling from Graphical Analysis of PET Data. *Journal of Cerebral Blood Flow Metabolism,* 1986, vol. 16, 834-840
- **A. BERTOLDO ; G. SPARACINO ; C. COBELLI.** Population Approach Improves Parameter Estimation of Kinetic Models from Dynamic PET Data. *IEEE Transactions on Medical Imaging,* 2004, vol. 23 (3), ISSN 0278-0062, 297-306
- **R. C. GONZALEZ ; R. E. WOODS.** Digital Image Processing. Prentice Hall
- **LINDSAY I SMITH.** *A tutorial on Principal Components Analysis,* 26 February 2002, http://www.cs.otago.ac.nz/cosc453/student_tutorials/principal_components.pdf
- **PASHA RAZIFAR.** Novel Approaches for Application of Principal Component Analysis on PET Images for Improvement of Image Quality and Clinical Diagnosis. *PhD thesis,* ISSN 1651-6214, ISBN 91-554-6387-8
- **C. S. PATLAK ; R. G. BLASBERG.** Graphical Evaluation of Blood-to-Brain Transfer Constants from Multiple-Time Uptake Data. *Journal of Cerebral Blood Flow Metabolism,* 1985, vol. 5, 584-590
- **RAZIFAR et al.** A new application of pre-normalized principal component analysis for improvement of image quality and clinical diagnosis in human brain PET studies. *Neuroimage,* 2006, vol. 33 (2), 588-598
- Application of Multivariate Image Analysis in Nuclear Medicine: Principal Component Analysis (PCA) on Dynamic Human Brain Studies with Positron Emission Tomography (PET) for Discrimination of Areas of Disease at High Noise Levels. **RAZIFAR ; BERGSTRÖM.** Techniques and Applications of Hyperspectral Image Analysis. John Wiley & Sons, 2007, 313-334
- Performance evaluation of principal component analysis in dynamic FDG-PET studies of recurrent colorectal cancer. **THIREOU et al.** Computerized Medical Imaging and Graphics. Elsevier, 2002, 43-51